# EUROPEAN PATENT APPLICATION

(11) **EP 4 656 145 A1**
(43) Date of publication of application: **03.12.2025**
(21) Application number: 23923748.0
(22) Date of filing: 07.11.2023
(51) Int. Cl.: A61B 17/12

(54) **SPRING COIL CONVEYING DEVICE AND SPRING COIL SYSTEM**

(30) Priority: 24.02.2023 CN 202310171624
(71) Applicant: Shanghai MicroPort Medical (Group) Co., Ltd., Shanghai 201203 (CN)
(72) Inventor: GU, Jialei, Shanghai 201203 (CN); LIU, Wei, Shanghai 201203 (CN); ZHU, Jing, Shanghai 201203 (CN); WANG, Xueqin, Shanghai 201203 (CN); YUE, Bin, Shanghai 201203 (CN)
(74) Representative: Marks & Clerk LLP
(86) International application number: PCT/CN2023/130163
(87) International publication number: WO 2024/174584

(57) **Abstract**

The present invention relates to a spring coil conveying device and a spring coil system. The spring coil system includes a spring coil and a spring coil conveying device. The conveying device includes a push rod and a manipulation member. A notch is provided in an outer circumference of the push rod at a distal end thereof, and the manipulation member is disposed inside the push rod so as to extend along an axis thereof. A proximal end of the manipulation member is located at a proximal end of the push rod, and the manipulation member distally extends along the axis of the push rod to the notch. A spring coil connector is looped through the notch in the conveying device and confined therein by the manipulation member, thereby attaching the spring coil to the conveying device. When a force is applied to the manipulation member to cause it to displace toward the proximal end of the push rod and thereby no longer confine the connector, the connector is allowed to dislodge from the notch, resulting in its detachment. The present invention overcomes the problems of a complex mechanical design and highly challenging fabrication with conventional mechanical detachment approaches.

## Description

### TECHNICAL FIELD

The present invention relates to the field of medical devices and, in particular, to spring coil conveying device and a spring coil system.

### BACKGROUND

Spring coils are implants for closing arterial fistulas and aneurysms. They are also commonly used for vascular closure in emergency cases of accidental vascular trauma and hemorrhage during surgical procedures. Spring coils achieve closure by slowing down local blood flow and thereby promoting thrombosis there.

Accurate delivery and release of a spring coil to a target lesions site in a patient's body require the use of an associated conveying device, which is a separate component and must be detached from the spring coil once the latter is successfully implanted. Such detachment may be accomplished mechanically, electrolytically, thermally, hydraulically or otherwise. Electrolytic detachment and thermal detachment may create a large current and high temperature at the lesion, possibly leading to local thrombosis or vascular trauma. Hydraulic detachment involves complex operations. Moreover, many existing detachment designs (including electrolytic, thermal and hydraulic) rely on the use of a specialized detacher, which leads to not only a complex detachment process lacking robustness and associated with a higher risk of failure but also an increase in the economic burden of the patient. Mechanical detachment is advantageous in not requiring additional use of a current input or electrolytic solution, rapid detachment and reliable connection, and is therefore widely adopted by spring coil products on the current market.

Most of these existing products employ, or are based on, the mechanical detachment designs of Medtronic and Boston Scientific, which mostly rely on a snap engagement mechanism in the form of a ball joint or male/female pair. However, such designs are complex in structure, and the spring coil connector and the socket, from which it is to be detached, require extremely high-precision processing. On the other hand, since the spring coil and the conveying device are small in size, higher processing precision would mean greater challenges placed on the manufacturing. Therefore, the fabrication is complex and expensive. There are also degradable spring coils available on the market, which are another type of popular product. However, when used with mechanical detachment, a spring coil connector would be necessary for such a degradable spring coil, which is, however, non-degradable. As a consequence, the spring coil would have at least one component remaining even after the lesion heals. Therefore, the existing mechanical detachment approaches are almost inapplicable to degradable spring coils due to poor compatibility.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY

It is an object of the present invention to provide a spring coil conveying device and a spring coil system, which overcome the problems with existing mechanical detachment techniques, including a complex mechanical design and highly challenging fabrication.

To this end, the present invention provides a spring coil conveying device including:
a push rod, an outer circumference of a distal end of the push rod provided with a notch; and
a manipulation member disposed in the push rod and extending along an axis of the push rod, a proximal end of the manipulation member located at a proximal end of the push rod, a distal end of the manipulation member extending along the axis of the push rod to the notch,
wherein the conveying device is configured so that a connector of the spring coil is looped through the notch and confined in the notch by the manipulation member, and
the conveying device is also configured so that when the manipulation member is stressed to move towards the proximal end of the push rod and release the confining of the manipulation member on the connector, the connector is allowed to dislodge from the notch to complete its detachment.

Optionally, at least one of the proximal and distal ends of the manipulation member may be attached to the push rod, wherein in the case of the distal end of the manipulation member being attached to the push rod, the distal end of the manipulation member is detachably attached to the distal end of the push rod.

Optionally, the push rod may include a rod body and a flexible tip mounted at a distal end of the rod body, wherein the notch is provided in an outer circumference of a distal end of the rod body, and the manipulation member is disposed inside the rod body and extends along an axis of the rod body, the distal end of the manipulation member axially extends through the notch and is attached to a proximal end of the flexible tip in a detachable manner.

Optionally, the flexible tip may be provided with a stop recess in its proximal end face, in which the distal end of the manipulation member is received.

Optionally, the rod body may include a distal body section and a proximal body section, which are axially joined to each other, wherein the distal body section is a polymer tube for medical use; the proximal body section is a polymer tube or a metal tube for medical use; and the flexible tip is mounted at a distal end of the distal body section.

Optionally, the conveying device may further include a foolproof detachment rod and a foolproof member, the foolproof detachment rod disposed at the proximal end of the push rod and attached to the push rod, wherein the proximal end of the manipulation member is attached to the foolproof detachment rod, and the conveying device is configured so that the foolproof member is able to be manipulated to detach the foolproof detachment rod from the push rod and that once detached from the push rod, the foolproof detachment rod is stressed to drive the manipulation member to move toward the proximal end of the push rod.

Optionally, the foolproof detachment rod may be attached to the push rod through the foolproof member, the foolproof detachment rod is detachable from the push rod by manipulating the foolproof member, or wherein the foolproof detachment rod has a proximal portion and a distal portion, the foolproof member is disposed between the proximal portion and the distal portion of the foolproof detachment rod, the distal portion of the foolproof detachment rod attached to the push rod, the proximal portion of the foolproof detachment rod attached to the proximal end of the manipulation member, wherein the conveying device is configured so that the foolproof member is able to be manipulated to detach the proximal portion from the distal portion and hence detach the proximal portion from the push rod.

Optionally, the attachment of the proximal end of the manipulation member to the foolproof detachment rod may be accomplished by at least one of a heat shrink tube, welding and gluing. Alternatively or additionally, the foolproof detachment rod may be fitted over the outer circumference of the push rod at the proximal end thereof.

Optionally, the foolproof member may be provided as a preformed weakened region of the foolproof detachment rod, which is configured not to break when the foolproof detachment rod is pulled and to break when the foolproof detachment rod is twisted to separate the proximal portion of the foolproof detachment rod from the distal portion thereof.

Optionally, the preformed weakened region may contain multiple hollow structures spaced around a circumference of the foolproof detachment rod.

Optionally, a distal endpoint of the notch may be spaced from a distal endpoint of the push rod at a distance of 0.5 mm to 2.0 mm.

Optionally, the notch may include a distal bevel and a proximal step, the distal bevel forming an acute angle with a positive direction defined along the axis of the push rod, the proximal step forming a right, acute or obtuse angle with the positive direction along the axis of the push rod.

Optionally, the manipulation member may be made from a metal wire for medical use.

To the above end, the present invention also provides a spring coil system including a spring coil and the spring coil conveying device as defined above, the spring coil including a spring coil body and a connector attached to the spring coil body, the connector looped through the notch in the conveying device and confined therein by the manipulation member of the conveying device.

Optionally, the spring coil may be a degradable spring coil.

The present invention provides a spring coil conveying device including: a push rod distally provided with a notch in an outer circumference thereof; and a manipulation member disposed inside the push rod so as to extend along an axis thereof, the manipulation member having a proximal end located at a proximal end of the push rod, the manipulation member distally extending along the axis of the push rod to the notch, wherein the conveying device is configured so that a spring coil connector is looped through the notch and confined therein by the manipulation member, and the conveying device is also configured so that when a force is applied to the manipulation member to cause it to displace toward the proximal end of the push rod and thereby no longer confine the connector, the connector is allowed to dislodge from the notch, resulting in its detachment.

With this arrangement, the spring coil can be mechanically detached without using a specialized detacher, allowing for a simpler detachment process involving less complex operation and associated with a lower risk of failure. In particular, the mechanical detachment design is simple in structure and allows for easy fabrication with less challenging processing of the push rod and the spring coil connector. Thus, the processing and fabrication can be achieved at lower cost. In particular, the spring coil is fully degradable without any component remaining in a patient's body, making the mechanical detachment design compatible for use with all types of spring coils.

As the spring coil system of the present invention is based on the same inventive concept as the spring coil conveying device of the present invention and thus has all the advantages of the latter, the advantages thereof are not repeated herein.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 schematically illustrates a spring coil system according to an embodiment of the present invention, in which a spring coil is connected to a conveying device;
Fig. 2 is a partial cross-sectional view of a distal portion of a conveying device according to an embodiment of the present invention, showing a spring coil, which has not been detached from the conveying device yet;
Fig. 3a schematically illustrates a notch according to an embodiment of the present invention, which has a non-linear proximal step;
Fig. 3b schematically illustrates a notch according to another embodiment of the present invention, which has a linear proximal step;
Fig. 4 schematically illustrates a proximal portion of a conveying device according to an embodiment of the present invention, which remains attached to a spring coil but has not been detached therefrom yet;
Fig. 5 is a cross-sectional view of a proximal portion of a conveying device according to an embodiment of the present invention, which remains attached to a spring coil but has not been detached therefrom yet;
Figs. 6a and 6d are schematic circumferentially rolled-out views of a foolproof member according to an embodiment of the present invention.

List of Reference Numerals
1 push rod; 11 rod body; 111 distal body section; 112 proximal body section; 12 flexible tip; 13 stop recess; 14 foolproof detachment rod; 141 distal attachment zone; 142 proximal attachment zone; 15 foolproof member; 151 hollow structure; 2 connector; 3 manipulation member; 4 spring coil body; 5 notch; 51 distal bevel; 52 proximal step.

### DETAILED DESCRIPTION

Various exemplary embodiments are described below. Reference is made to these examples in a non-limiting sense, as it should be noted that they are provided to illustrate more broadly applicable aspects of the devices, systems and methods. Various changes may be made to these embodiments and equivalents may be substituted without departing from the true spirit and scope of the various embodiments. In addition, many modifications may be made to adapt a particular situation, material, composition of matter, process, process act(s) or step(s) to the objective(s), spirit or scope of the present invention. All such modifications are intended to be within the scope of the claims made herein.

Any dimensions mentioned in the foregoing general description or the following detailed description are exemplary only, and should not be taken as limiting the subject matter of the present invention, unless so indicated in various exemplary embodiments. In addition, the various configurations of the embodiments described herein are mutually supplementary, but not purely alternative, unless so stated. In other words, a configuration of one embodiment can be freely combined with configuration(s) of other embodiment(s), as would be readily understood by those of ordinary skill in the art, unless they are explicitly described as being alternative to each other or one another.

As used herein, the terms "proximal" and "distal" describe relative orientations, positions and directions of components of a conveying device or spring coil system or actions taken thereon, as viewed by an operator operating the medical device. Without wishing to be limiting, "proximal" usually refers to an end of the conveying device or spring coil system closer to the operator, and "distal" to an end that enters the body of a patient first, during normal operation of the device or system. In the context herein, "distal" and "proximal" refer to relative locations, rather than particular ends. For example, a "distal end" of a push rod refers to a location near the distal end of the push rod, rather than exactly the distal end. When used herein to describe a push rod, the term "axial" refers to a direction along a central axis thereof, "circumferential" to a direction about the central axis, and "central axis" to a lengthwise direction of the push rod.

The present invention will be described in greater detail below with reference to the accompanying drawings, which illustrate preferred embodiments thereof. Should there be no conflict, the embodiments described hereunder and features thereof can complement or be combined with each other or one another.

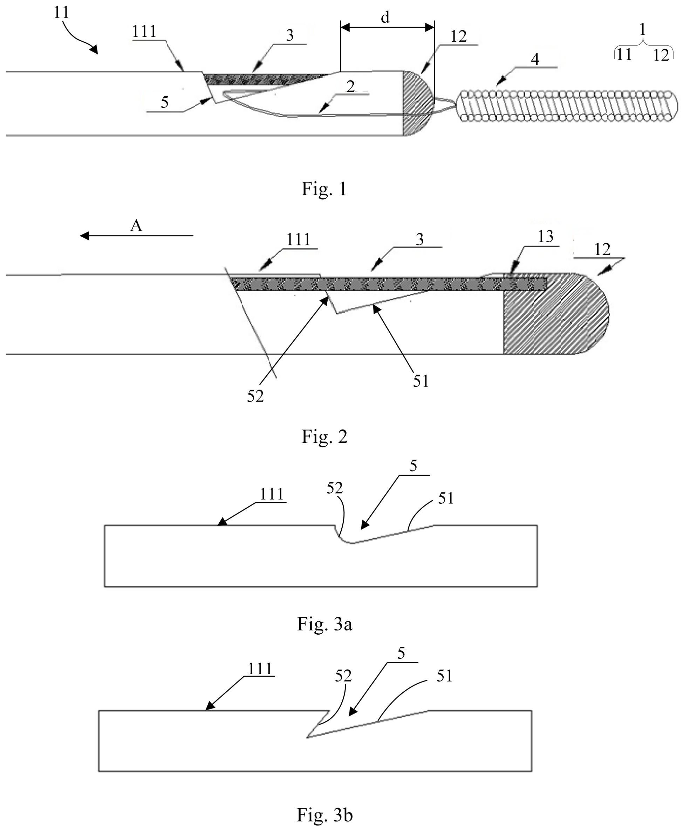

Referring to Figs. 1 and 2, the present invention provides a spring coil system including a spring coil and an associated spring coil conveying device (referred hereinafter as the "conveying device" for short). The conveying device is used to accurately deliver a spring coil to a lesion site and release the spring coil thereat. Once the spring coil is successfully implanted, the conveying device is detached from the spring coil. According to the present invention, the spring coil may be a degradable or non-degradable spring coil, with a degradable spring coil being more preferred.

The conveying device includes a push rod 1 and a manipulation member 3. The spring coil includes a connector 2 and a spring coil body 4. The connector 2 is directly connected to the spring coil body 4. That is, the connector 2 forms part of the spring coil itself. The push rod 1 is distally provided with a notch 5 in an outer circumference thereof. The manipulation member 3 is disposed within the push rod 1 so as to extend along an axis of the push rod 1. A proximal end of the manipulation member 3 is located at a proximal end of the push rod 1, and a distal end of the manipulation member 3 extends along an axis of the push rod 1 to the notch 5. Here, by "a distal end of the manipulation member 3 extends along an axis of the push rod 1 to the notch 5", it is meant that the manipulation member 3 may distally extends along the axis through the entire notch 5, or only into the notch 5 without reaching the distal end of the notch 5 or a more distal location outside it. The spring coil connector 2 may be looped through the notch 5 of the conveying device and restricted within the notch 5 by the manipulation member 3. In order to avoid dislodgement of the connector 2 from the notch 5, it is necessary for the manipulation member 3 to extend to the notch 5. In this way, the connector 2, the notch 5 and the manipulation member 3 can work together to connect the conveying device to the spring coil. Once connected to the spring coil, the conveying device may be used to deliver the spring coil to a lesion site. Moreover, after the spring coil is successfully released and implanted, the conveying device may be detached from the spring coil.

In this embodiment, the detachment of the conveying device is mechanically detached from the spring coil. The mechanical detachment may be accomplished by displacing the manipulation member 3 toward the proximal end of the push rod 1 through directly or indirectly manipulating the proximal end of the manipulation member 3 until the manipulation member 3 no longer restricts the connector 2 and allows the connector 2 to be moved out of the notch 5, thereby achieving the detachment.

As would be appreciated, the connector 2 includes a loop, which may be directly looped through the notch 5, and the loop is restricted by the manipulation member 3 within the notch 5 without dislodging from the notch 5. That is, the notch 5 and the manipulation member 3 together form a closed loop, in which the loop is confined. Therefore, the spring coil system of the present invention utilizes double-loop locking to lock and detach the spring coil to and from the conveying device. This mechanical detachment arrangement is simple in structure and dispenses with the need for the use of an additional detacher, providing ease of use and robust and reliable detachment. Moreover, according to the embodiment, all the components involved (including the notch 5, the connector 2 and the manipulation member 3) can be fabricated through simple processes without using separately customized molds for high-precision processing. Therefore, it is easy to implement in terms of manufacturing and requires non-expensive processing and fabrication. Moreover, it provides a reliable detachable connection, and the detachment can be accomplished very easily with high robustness and high efficiency. In particular, the mechanical detachment arrangement of this embodiment can be compatibly used with a degradable spring coil. In this case, the entire spring coil, including the connector 2, is degradable, without any component remaining after the lesion heals. Therefore, this mechanical detachment arrangement is highly suitable for use with a degradable spring coil. It will be understood that it is unsuitable for conventional spring coil connectors to be made of a degradable material due to inadequate mechanical strength of such materials. On the other hand, snap engagement-based connectors must have a very small size, and therefore when made of a degradable material, would not be able to guarantee sufficient connection strength and impose even higher processing challenges.

The manipulation member 3 functions mainly to confine the connector 2 within the notch 5 and prevent its dislodgement from the notch 5. Accordingly, in practical use, it is desirable to minimize displacement of the manipulation member 3 as the spring coil delivery is moving, because such displacement may lead to premature detachment of the connector 2. To this end, at least one of the proximal and distal ends of the manipulation member 3 is attached to the push rod 1 to reduce the likelihood of displacement of the manipulation member 3 during movement of the spring coil delivery. In the case of the distal end of the manipulation member 3 being attached to the push rod 1, the attachment between the distal end of the manipulation member 3 and the push rod 1 is detachable. Here, by "the attachment is detachable", it is intended to mean that even after the distal end of the manipulation member 3 is attached to the distal end of the push rod 1, the distal end of the manipulation member 3 can still be detached from the push rod 1 to enable detachment of the connector 2. In the case of the proximal end of the manipulation member 3 being attached to the proximal end of the push rod 1, this attachment between the proximal end of the manipulation member 3 and the proximal end of the push rod 1 is usually non-detachable and may be accomplished, for example, by gluing, welding or a heat shrink tube. Moreover, in the case of the proximal end of the manipulation member 3 being attached to the proximal end of the push rod 1, in addition to restricting the manipulation member 3, the push rod 1 can be directly manipulated at the proximal end to indirectly cause proximal retraction of the manipulation member 3. It should be noted that, as would be appreciated by those skilled in the art, the present invention is not limited to any particular method for creating the detachable attachment between the distal end of the manipulation member 3 and the distal end of the push rod 1. Optionally, the distal end of the manipulation member 3 may be attached to the distal end of the push rod 1, for example, by a male/female pair, threads, snap engagement, etc.

Preferably, the manipulation member 3 is attached to the push rod 1 at both the proximal and distal ends, because this can better prevent displacement of the manipulation member 3, effectively reducing the risk of premature detachment of the connector 2. Although it has been described above that displacement of the manipulation member 3 relative to the push rod 1 can be reduced by attaching at least one end of the manipulation member 3 to the push rod 1, it is contemplated herein that the manipulation member 3 may be manually held against displacement. Moreover, in order to detach the connector 2, the manipulation member 3 may be manually retracted proximally relative to the push rod 1.

In order to achieve robust and efficient detachment, the manipulation member 3 is desired to exhibit good force transfer properties and good tensile resistance during use. To this end, the manipulation member 3 may be made of a common metallic material for medical use, preferably an elastic metallic material for medical use, examples of which may include, but are not limited to stainless steel and nickel-titanium alloy. Considering that the push rod 1 is small in size, the manipulation member 3 is desired to have a compatible small size, and the manipulation member 3 may be therefore made of a metal wire commonly used in the medical field, such as a stainless steel or nickel-titanium alloy wire. In this case, the metal wire can be simply inserted into the push rod 1 so as to extend into the notch 5 to restrict the connector 2. This mechanical design is simple and inexpensive. It will be understood that either a single or multiple such metal wires may be used to restrict the connector 2, without departing from the scope of this application. In general, a single metal wire would suffice.

As shown in Fig. 1, the notch 5 is usually formed at a predetermined location spaced at a distance from the distal end of the push rod 1. Hereinafter, this predetermined location is referred to as a detachment zone. A distance d from a distal endpoint of the notch 5 to a distal endpoint of the push rod 1 needs to be appropriately determined. If the distance d is too large, the connector 2 may be too long to enable accurate spring coil release. On the other hand, if the distance d is too small, the connector 2 may be more likely to dislodge. For these reasons, the distance d from the distal endpoint of the notch 5 to the distal endpoint of the push rod 1 is preferred to be 0.5 mm to 2.0 mm. This application is not limited to any particular method for forming the notch 5. As will be understood, the notch 5 may be formed in the outer circumference of the push rod 1 at the distal end thereof using various techniques, such as laser cutting, chemical etching, mechanical cutting or integral forming.

The notch 5 may assume any of various suitable shapes, and the present application is not limited to any particular such shape, as long as it can ensure that, as a result of the manipulation member 3 being retracted toward the proximal end of the push rod 1, the connector 2 will smoothly move distally out of the push rod 1, rather than being tensioned and deformed at the proximal end thereof, leading to a delivery failure. It will be understood that the notch 5 may be a slit, a notch or a hollow structure larger than both.

As shown in Fig. 2, in this embodiment, the notch 5 has a distal bevel 51 and a proximal step 52. The distal bevel 51 is located at a distal end of the proximal step 52. The distal bevel 51 forms an acute angle with a positive direction A defined along the axis of the push rod 1 and pointing from the distal end of the push rod 1 to the proximal end thereof. The connector 2 can slide on the distal bevel 51 toward the distal end of the push rod 1 and thereby more smoothly move out of the notch 5. The distal bevel 51 may be linear or non-linear. The distal bevel 51 is preferred to be gentle and smooth to reduce resistance to detachment. The proximal step 52 of the notch 5 can prevent, to some extent, displacement of the connector 2 toward the proximal end of the push rod 1. The proximal step 52 may form an acute, obtuse or right angle with the positive direction A along the axis of the push rod 1. The proximal step 52 may be linear or non-linear. The notch 5 may have a depth on a cross section of the push rod 1, which is determined as desired. Desirably, the depth of the notch 5 enables the push rod have sufficient distal strength, which can prevent distal breakage of the push rod at the notch 5. There may be an arcuate or non-arcuate transition between the distal bevel 51 and the proximal step 52.

Fig. 3a shows a structural example of the notch 5 according to an embodiment of the present invention, in which the proximal step 52 is a non-linear step forming an obtuse angle with the positive direction along the axis of the push rod 1, and the distal bevel 51 is a linear bevel. Fig. 3b shows another structural example of the notch 5 according to an embodiment of the present invention, in which the proximal step 52 is a linear step forming an acute angle with the positive direction along the axis of the push rod 1, and the distal bevel 51 is a linear bevel. However, it will be understood that the present invention is not limited to the structural examples of notch 5 shown in the figures.

As shown in Figs. 1 and 2, the connector 2 may be made of a common polymeric material for medical use, preferably, the connector 2 is made of a degradable polymeric material for medical use, examples of which may include, but are not limited to polylactic acid, polycaprolactone, poly(glycolide-co-lactide) and poly(p-dioxanone). Moreover, it may be made of one or more of such polymeric materials for medical use. The connector 2 may be any selected from suitable linear connectors, which are simple in structure, easy to process and manufacture and small in size, such as wires, threads, yarns and bands. The connector 2 is desired to have good tensile resistance, which makes it resistant to breakage during use. The spring coil body 4 may be selected as a conventional implantable spring coil, without limiting the application in any sense.

Referring to Figs. 1 and 2, in one embodiment, the push rod 1 includes a rod body 11 and a flexible tip 12. The flexible tip 12 is mounted at a distal end of the rod body 11, and the notch 5 is formed in an outer circumference of the rod body 11 at a distal end thereof. The manipulation member 3 is disposed inside the rod body 11 so as to extend along an axis of the rod body 11. The manipulation member 3 may axially extend distally through the notch 5 and be detachably coupled to a proximal end of the flexible tip 12. The present invention is not limited to any particular method for creating the detachable coupling. With this arrangement, the flexible tip 12 can be directly used to restrict the distal end of the manipulation member 3 to prevent premature detachment of the connector 2.

The flexible tip 12 may be a non-invasive structure made of a flexible material. An outer diameter of the flexible tip 12 may be substantially equal to an outer diameter of the rod body 11 at the distal end thereof. The flexible tip 12 enables the distal end of the push rod 1 not only to cause less damage to a blood vessel but also to restrict the manipulation member 3. However, the present invention is not so limited, because as would be appreciated by those skilled in the art, in alternative embodiments, the flexible tip 12 may be omitted, and the distal end of the manipulation member 3 may be instead restricted directly by the rod body 11 itself.

The flexible tip 12 may be made essentially of a flexible, elastic material for medical use, examples of which may include, but are not limited to, silicone, hydrogel and TPU. Moreover, it may be made of one or more of such materials. Preferably, the flexible tip 12 contains a radiopaque contrast material, examples of which may include, but are not limited to, barium sulfate, tantalum powder, iodine-based contrast media, tungsten powder and other contrast materials. The flexible tip 12 may have a smooth spherical surface, which can reduce possible damage to a blood vessel. The flexible tip 12 may be attached to the distal end of the manipulation member 3 in any suitable manner. For example, the distal end of the manipulation member 3 may be inserted into a stop recess 13 in a proximal end face of the flexible tip 12. Alternatively, a stop stud on the proximal end face of the flexible tip 12 may be inserted into a stop recess in a distal end face of the manipulation member 3. Still alternative approaches for preventing displacement of the manipulation member 3 are also possible. In the present invention, a stop recess 13 is provided in the proximal end face of the flexible tip 12 to reduce displacement of the manipulation member 3 during delivery, which is associated with a risk of causing premature detachment of the connector 2. It will be understood that the stop recess 13 is a blind recess. The present invention is not limited to any particular method for forming the blind recess, and possible examples thereof may include cutting, punching, etching and integral forming. The stop recess 13 may have an inner diameter, which is 1.0-1.5 times an outer diameter of the manipulation member 3.

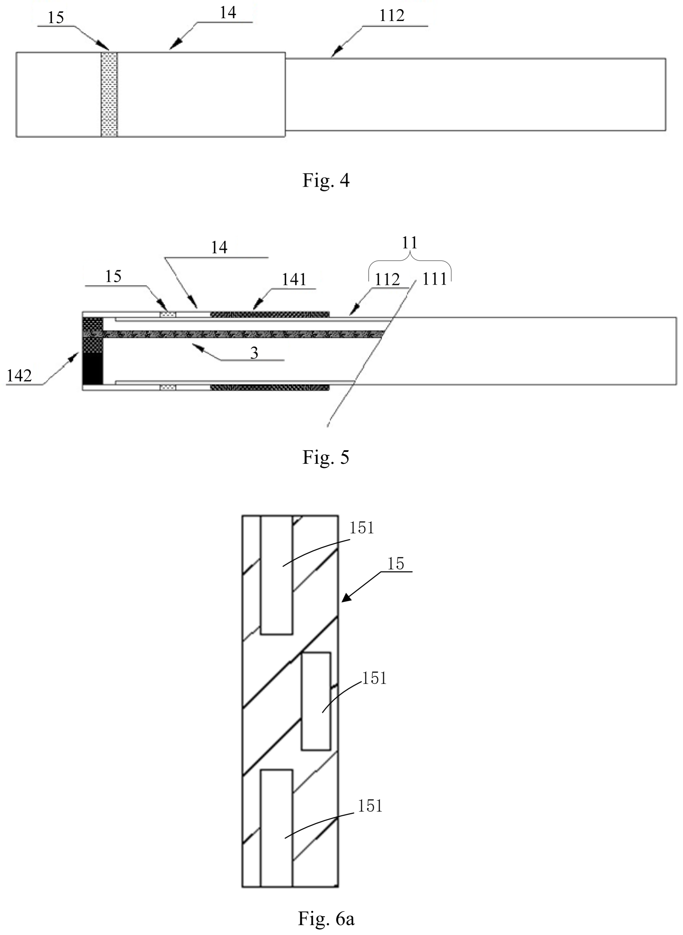

The rod body 11 may include a distal body section 111 and a proximal body section 112, which are joined along an axis thereof (see Figs. 4 and 5). The flexible tip 12 may be mounted at a distal end of the distal body section 111.

Preferably, the distal body section 111 is a medical polymer tube made of a common polymeric material for medical use, which enables the distal end of the push rod to be sufficiently flexible and safe for the human body. Of course, it is also contemplated herein that the distal body section 111 is made of a non-polymeric material. In this embodiment, the distal body section 111 is a medical polymer tube, which may be made of, as a non-limiting example, one or more selected from polytetrafluoroethylene, polyvinylpyrrolidone, polyamides and polyimides. Preferably, the distal body section 111 is a plastic tube, which allows for easy formation of the notch 5 in the distal body section 111, in contrast to a spring coil spring obtained by winding a metal wire, as is conventional.

Referring to Figs. 4 to 5, a proximal end of the distal body section 111 is joined to a distal end of the proximal body section 112 so that the two are coaxial with each other. The proximal body section 112 and the distal body section 111 may be made of the same or different materials. The rod body 11 is desired to provide certain mechanical properties, and a good trade-off is necessary between its delivery performance and compliance to allow the rod body 11 to be mechanically strong enough to be advanced through a microcatheter or catheter sheath while being compliant enough to be advanced through a tortuous vessel segment, as required for successfully delivering the spring coil to a target lesion site. Generally, the proximal body section 112 is stiffer than the distal body section 111, and the distal body section 111 is more flexible than the proximal body section 112. The proximal body section 112 may be implemented as a medical polymer or metal tube, examples of which may include, and are not limited to, a stainless steel tube, nickel-titanium alloy tube, a polytetrafluoroethylene tube, a silicone tube and a composite tube.

As shown in Figs. 4 and 5, in one embodiment, the conveying device further includes a foolproof detachment rod 14 and a foolproof member 15. The foolproof detachment rod 14 is disposed at the proximal end of the push rod 1 and attached to the push rod 1. In this embodiment, the foolproof detachment rod 14 is attached to the proximal body section 112 of the rod body 11. Additionally, the manipulation member 3 is proximally attached to the foolproof detachment rod 14. With this arrangement, the manipulation member 3 is indirectly coupled to the push rod 1 through the foolproof detachment rod 14. After the foolproof detachment rod 14 is detached from the push rod 1, it can be manipulated to directly proximally pull the manipulation member 3. In this case, the conveying device is configured so that the foolproof member 15 can be manipulated to detach the foolproof detachment rod 14 from the push rod 1. Once detached from the push rod 1, the foolproof detachment rod 14 can be proximally pulled to retract the manipulation member 3 toward the proximal end of the push rod 1 displacement. The foolproof detachment rod 14 and the foolproof member 15 enable even more reliable coupling between the spring coil and the conveying device and even more reliable, easier, more convenient and more robust detachment of them from each other. In one embodiment, the foolproof detachment rod 14 may be disposed over the outer circumference of the rod body 11 at the proximal end thereof, for example, over an outer circumference of the proximal body section 112 at a proximal end thereof. In other embodiments, the rod body 11 may proximally nested over the foolproof detachment rod 14, while still obtaining the substantially same benefits.

In some embodiments, the foolproof detachment rod 14 may be directly coupled to the rod body 11 through the foolproof member 15. Moreover, the foolproof member 15 may be manipulated to couple the foolproof detachment rod 14 to the rod body 11, or detach them from each other. Optionally, the foolproof member 15 may be a mechanical locking means, which can couple the foolproof detachment rod 14 to the rod body 11 or detach them from each other by means mechanical locking such as threaded connection or snap engagement. In other embodiments, the foolproof detachment rod 14 includes a proximal portion and a distal portion. The distal portion of the foolproof detachment rod 14 is directly attached to the rod body 11, and the proximal portion of the foolproof detachment rod 14 is directly attached to the proximal end of the manipulation member 3. In such cases, the conveying device may be configured so that the foolproof member 15 can be manipulated to detach the proximal and distal portions of the foolproof detachment rod 14 from each other. Moreover, the foolproof member 15 may be disposed directly on the foolproof detachment rod 14, particularly, the foolproof member 15 is disposed between the proximal and distal portions of the foolproof detachment rod 14, and the foolproof member 15 can be destroyed to cause detachment of the two sections of the foolproof detachment rod 14 from each other. After the detachment of the two sections of the foolproof detachment rod 14 takes place, the distal portion of the foolproof detachment rod 14 remains attached to the rod body 11, and the proximal portion of the foolproof detachment rod 14 remains attached to the manipulation member 3. At this point, since the proximal portion of the foolproof detachment rod 14 has been decoupled from the push rod 1, the proximal portion of the foolproof detachment rod 14 can be manipulated to drive the manipulation member 3 to proximally move relative to the push rod 1.

In the present embodiment, in each case, the foolproof detachment rod 14 is coupled to the proximal body section 112 or detached therefrom by manipulating the foolproof member 15. Only when the foolproof detachment rod 14 has been disconnected from the proximal body section 112, can the foolproof detachment rod 14 be proximally pulled to cause the manipulation member 3 to proximally move therewith until the manipulation member 3 does not restrict the connector 2 any longer. In alternative embodiments, the foolproof detachment rod 14 and the foolproof member 15 may be omitted, and the manipulation member 3 may be instead directly retracted by manipulating the proximal end of the manipulation member 3. However, in the present embodiment, through providing the foolproof detachment rod 14 and the foolproof member 15, safer and reliable mechanical detachment can be achieved.

The foolproof detachment rod 14 may be made of a common polymeric material for medical use, e.g., one or more of polytetrafluoroethylene, polyvinylpyrrolidone, polyamides, polyimides, polycarbonates and polyolefins.

In an embodiment, as shown in Fig. 5, the distal portion of the foolproof detachment rod 14 is directly attached to the proximal body section 112, and they together form a distal attachment zone 141. The attachment of the distal portion of the foolproof detachment rod 14 to the proximal body section 112 may be accomplished by at least one of a heat shrink tube, welding and gluing. In an embodiment, as shown in Fig. 5, the proximal portion of the foolproof detachment rod 14 is attached to the proximal end of the manipulation member 3, and they together form a proximal attachment zone142. Similarly, the attachment of the proximal portion of the foolproof detachment rod 14 to the manipulation member 3 may be accomplished by at least one of a heat shrink tube, welding and gluing. In view of a small size of the push rod 1, the proximal end of the manipulation member 3 is preferably glued to the proximal portion of the foolproof detachment rod 14, from the point of view of ease of implementation in terms of manufacturing.

It will be understood that, in this embodiment, the foolproof member 15 has a locking configuration and an unlocking configuration. In the locking configuration, the foolproof member 15 maintains the proximal body section 112 attached to the foolproof detachment rod 14 without easy separation and prevents the separation between the proximal body section 112 and the foolproof detachment rod 14. When the foolproof member 15 is in the unlocking configuration, the proximal body section 112 is separated from the foolproof detachment rod 14, and the proximal body section 112 is movable relative to the foolproof detachment rod 14 and thus can be manipulated to retract the manipulation member 3 with foolproof detachment rod 14. The foolproof member 15 ensures that the manipulation member 3, the foolproof detachment rod 14 and the rod body 11 will not displace relative to one another during delivery and release of spring coil, preventing premature detachment of the spring coil that may occur due to such relative displacement. Once the spring coil is released in place, the foolproof member 15 is put into the unlocking configuration, allowing the manipulation member 3 to be retracted with the foolproof detachment rod 14 to result in spring coil detachment.

Those skilled in the art can devise many designs to implement the foolproof member 15, including but not limited to, threaded locks, snap locks and preformed weakened region. The foolproof member 15 may be implemented as a threaded lock, which threadedly locks and attaches the foolproof detachment rod 14 to the proximal body section 112. Alternatively, the foolproof member 15 may be implemented as a snap lock, which locks and attaches the foolproof detachment rod 14 to the proximal body section 112 by means of snap engagement. Still alternatively, the foolproof member 15 may be directly implemented as a preformed weakened region of the foolproof detachment rod 14, which can withhold pulling forces for causing displacement of the foolproof detachment rod 14, but will more easily break than the rest of the foolproof detachment rod 14 when the latter is twisted.

In this embodiment, the foolproof member 15 is located between the proximal and distal portions of the foolproof detachment rod 14, the foolproof member 15 is provided as a preformed weakened region of the foolproof detachment rod 14. The preformed weakened region may be implemented by various means including hollow structures 151, cracks, thinner wall portions, cornered edges and other types of defects. In one embodiment, as shown in Figs. 6a to 6d, the preformed weakened region includes multiple hollow structures 151 spaced around a circumference of the foolproof detachment rod 14. The present invention is not limited to any particular shape of the hollow structures 151. The hollow structures 151 significantly reduce circumferential strength of the foolproof detachment rod 14 while substantially maintaining its axial strength. For example, one or more of the hollow structures 151 may be formed around the circumference of the foolproof detachment rod 14 by any of various techniques such as laser cutting, chemical etching, mechanical cutting and integral forming.

For this design of the foolproof detachment rod 14 with the hollow structures 151, it is essential that there are straight axially extending portions of the foolproof detachment rod 14 between the hollow structures, which enable the foolproof detachment rod 14 to have axial strength that is high enough to prevent its breakage during delivery. Meanwhile, the hollow structures that are spaced around the circumference of the foolproof detachment rod 14 introduce some stress concentration points (e.g., the corners of hollow structures when they are rectangular or rhombic), which reduce the circumferential strength of the foolproof detachment rod 14 to such an extent that it is prone to breakage when circumferentially twisted, resulting in successful detachment. It will be understood that after the foolproof detachment rod 14 breaks at the foolproof member 15 into two pieces: a distal piece previously joined to a distal end of the foolproof member 15, which remains attached to the proximal body section 112; and a proximal piece previously joined to a proximal end of the foolproof member 15, which is separated from the proximal body section 112.

The foolproof member 15 is further described below with reference to Figs. 6a to 6d.

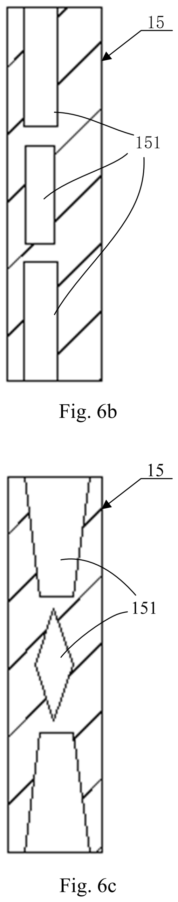

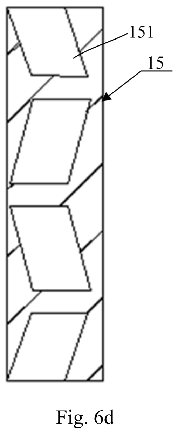

As shown in Fig. 6a, the hollow structures 151 may be rectangular in shape, and the hollow structures 151 are spaced around the circumference of the foolproof detachment rod 15, with adjacent hollow structures 151 being separated without mutual communication. The multiple hollow structures 151 may be of the same size or not, and may be spaced along the same or different circumferential lines. Fig. 6b also shows multiple rectangular hollow structures 151, which, however, differing from the circumferentially collinear arrangement shown in Fig. 6a, the hollow structures 151 are spaced along different circumferential lines. Fig. 6c shows otherwise-shaped hollow structures 151. For example, the hollow structures 151 may be trapezoidal or rhombic in shape. Moreover, these hollow structures 151 may be of the same or different shapes, and even in the former case, the hollow structures 151 may be of the same or different sizes. Fig. 6d shows hollow structures 151 in the shape of parallelograms. Of course, the hollow structures 151 are not limited to assume any of the enumerated shapes. Although the foolproof member 15 has been shown as having three or four hollow structures 151, the present invention is not so limited, as other numbers of hollow structures 151 are also possible. Further, the present invention is not limited to any particular relative positions of the hollow structures.

A detachment process for the spring coil system of the present embodiment is further described below in the context of the foolproof detachment rod 14 and the foolproof member 15 being included, as an example. In order to achieve detachment, the operator may circumferentially twist the foolproof detachment rod 14 at the proximal end of the push rod 1 until the foolproof member 15 breaks, separating the two portions of the foolproof detachment rod 14 from each other. After that, the operator may firmly hold the proximal end of the foolproof detachment rod 14 and retract it a distance (e.g., 1 to 2 cm), causing the manipulation member 3 attached to the proximal end of the foolproof detachment rod 14 to be retracted in the same way. As a result, the distal closed loop formed by the manipulation member 3 and the notch 5 will be opened up, unlocking the connector 2. The push rod 1 may be then retracted to cause the connector 2 to move on the distal bevel 51 of the notch 5 away from the push rod 1, thus achieving its detachment.

In the spring coil system of the present invention, the push rod provides sufficient pushability and twist resistance at its proximal end and sufficient flexibility at its push rod, which enables safe accessibility to a deep lesion. Once the spring coil is delivered to a target lesion, the push rod may be pushed and pulled back and forth to release the spring coil at a desired location and orientation. The spring coil may be detached and released after satisfactory packing has been attained. Compared with the prior art, the present invention dispenses with the use of a specialized detacher and allows for a simpler detachment process involving less complex operation and associated with a lower risk of failure. In particular, the mechanical detachment design is simple in structure and allows for easy fabrication with less challenging processing of the push rod and the spring coil connector. Thus, the processing and fabrication can be achieved at lower cost. In particular, the spring coil is fully degradable without any component remaining in a patient's body, making the mechanical detachment design compatible for use with all types of spring coils.

It should be noted that the embodiments disclosed herein are described in a progressive manner, with the description of each embodiment focusing on its differences from others. Cross reference can be made between the embodiments for their common or similar features. Since the system embodiments correspond to the method embodiments, they are described relatively briefly, and reference can be made to the method embodiments for more details thereof.

It also should be noted that while the invention has been described above with reference to preferred embodiments thereof, it is not limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent alternatives and modifications made to the foregoing embodiments based on the substantive disclosure of the invention without departing from the scope thereof fall within the scope.

It is also to be recognized that the terminology used herein is for the purpose of describing particular embodiments only, and is not intended to limit the scope of the present invention. It is noted that, as used herein and in the appended claims, the singular forms "a" and "an" include the plural reference unless the context clearly dictates otherwise. Thus, for example, a reference to "a step" or "a means" is a reference to one or more steps or means and may include sub-steps and sub-means. All conjunctions used are to be understood in the most inclusive sense possible. Thus, the term "or" should be understood as having the definition of a logical "or" rather than that of a logical "exclusive or" unless the context clearly necessitates otherwise. Further, implementation of the method and/or device according to the embodiments of the present invention may involve performing selected tasks manually, automatically, or a combination thereof.

## Claims

1. A conveying device for a spring coil, comprising:
a push rod, an outer circumference of a distal end of the push rod provided with a notch; and
a manipulation member disposed in the push rod and extending along an axis of the push rod, a proximal end of the manipulation member located at a proximal end of the push rod, a distal end of the manipulation member extending along the axis of the push rod to the notch,
wherein the conveying device is configured so that a connector of the spring coil is looped through the notch and confined in the notch by the manipulation member, and
the conveying device is also configured so that when the manipulation member is stressed to move towards the proximal end of the push rod and release the confining of the manipulation member on the connector, the connector is allowed to dislodge from the notch to complete its detachment.

2. The conveying device for the spring coil according to claim 1, wherein at least one of the proximal end and the distal end of the manipulation member is attached to the push rod, wherein in the case of the distal end of the manipulation member being attached to the push rod, the distal end of the manipulation member is detachably attached to the distal end of the push rod.

3. The conveying device for the spring coil according to claim 2, wherein the push rod comprises a rod body and a flexible tip, the flexible tip mounted at a distal end of the rod body, wherein the notch is provided in an outer circumference of a distal end of the rod body, and the manipulation member is disposed inside the rod body and extends along an axis of the rod body, the distal end of the manipulation member axially extends through the notch and is attached to a proximal end of the flexible tip in a detachable manner.

4. The conveying device for the spring coil according to claim 3, wherein a proximal end face of the flexible tip is provided with a stop recess, and wherein the distal end of the manipulation member is received in the stop recess.

5. The conveying device for the spring coil according to claim 3, wherein the rod body comprises a distal body section and a proximal body section, which are axially joined to each other, wherein the distal body section is a polymer tube for medical use; the proximal body section is a polymer tube or a metal tube for medical use; and the flexible tip is mounted at a distal end of the distal body section.

6. The conveying device for the spring coil according to claim 2, further comprising a foolproof detachment rod and a foolproof member, the foolproof detachment rod disposed at the proximal end of the push rod and attached to the push rod, wherein the proximal end of the manipulation member is attached to the foolproof detachment rod, and the conveying device is configured so that the foolproof member is able to be manipulated to detach the foolproof detachment rod from the push rod and that once detached from the push rod, the foolproof detachment rod is stressed to drive the manipulation member to move toward the proximal end of the push rod.

7. The conveying device for the spring coil according to claim 6, wherein the foolproof detachment rod is attached to the push rod through the foolproof member, the foolproof detachment rod is detachable from the push rod by manipulating the foolproof member, or wherein the foolproof detachment rod has a proximal portion and a distal portion, the foolproof member is disposed between the proximal portion and the distal portion of the foolproof detachment rod, the distal portion of the foolproof detachment rod attached to the push rod, the proximal portion of the foolproof detachment rod attached to the proximal end of the manipulation member, wherein the conveying device is configured so that the foolproof member is able to be manipulated to detach the proximal portion from the distal portion and hence detach the proximal portion from the push rod.

8. The conveying device for the spring coil according to claim 6, wherein the attachment of the proximal end of the manipulation member to the foolproof detachment rod is accomplished by at least one of a heat shrink tube, welding and gluing, and/or wherein the foolproof detachment rod is fitted over the outer circumference of the proximal end of the push rod.

9. The conveying device for the spring coil according to claim 7, wherein the foolproof member is provided as a preformed weakened region of the foolproof detachment rod, the preformed weakened region configured not to break when the foolproof detachment rod is pulled and to break when the foolproof detachment rod is twisted to separate the proximal portion of the foolproof detachment rod from the distal portion of the foolproof detachment rod.

10. The conveying device for the spring coil according to claim 9, wherein the preformed weakened region contains multiple hollow structures spaced around a circumference of the foolproof detachment rod.

11. The conveying device for the spring coil according to claim 1, wherein a distal endpoint of the notch is spaced from a distal endpoint of the push rod at a distance of 0.5 mm to 2.0 mm.

12. The conveying device for the spring coil according to claim 1, wherein the notch comprises a distal bevel and a proximal step, the distal bevel forming an acute angle with a positive direction defined along the axis of the push rod, the proximal step forming a right, acute or obtuse angle with the positive direction along the axis of the push rod.

13. The conveying device for the spring coil according to claim 1, wherein the manipulation member is made from a metal wire for medical use.

14. A spring coil system, comprising a spring coil and the conveying device for the spring coil of any one of claims 1 to 13, the spring coil comprising a spring coil body and a connector, the connector attached to the spring coil body, the connector looped through the notch in the conveying device, and the connector confined in the notch by the manipulation member of the conveying device.

15. The spring coil system according to claim 14, wherein the spring coil is a degradable spring coil.
